(19) Europäisches Patentamt
European Patent Office
Office européen des brevets

(11) Veröffentlichungsnummer: **0 339 442 A2**

(12) # EUROPÄISCHE PATENTANMELDUNG

(21) Anmeldenummer: **89106969.2**

(22) Anmeldetag: **19.04.89**

(51) Int. Cl.⁴: **C12N 1/20 , C12P 15/00 , C07C 39/12 , C07C 49/727 , A61K 31/12**

Patentansprüche für folgende Vertragsstaaten: ES + GR

Der Anmelder hat eine Erklärung nach Regel 28 (4) EPÜ (Herausgabe einer Probe nur an einen Sachverständigen) eingereicht. Eingangsnummer(n) der Hinterlegung(en): DSM 4357

(30) Priorität: **27.04.88 DE 3814217**

(43) Veröffentlichungstag der Anmeldung: **02.11.89 Patentblatt 89/44**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Grabley, Susanne, Dr.**
**Hölderlinstrasse 7**
**D-6240 Königstein Taunus(DE)**
Erfinder: **Wink, Joachim, Dr.**
**Bieberer Strasse 133**
**D-6050 Offenbach(DE)**
Erfinder: **Giani, Carlo, Dr.**
**Hedderichstrasse 69**
**D-6000 Frankfurt am Main 70(DE)**
Erfinder: **Seibert, Gerhard, Prof. Dr.**
**Gläserweg 21**
**D-6100 Darmstadt(DE)**
Erfinder: **Raether, Wolfgang, Dr.**
**Falkensteinstrasse 6**
**D-6072 Dreieich(DE)**
Erfinder: **Dobreff, Susanne**
**Otto-Lauffer-Strasse 2**
**D-3400 Göttingen(DE)**
Erfinder: **Zeeck, Axel, Prof. Dr.**
**Brüder-Grimm-Allee 22**
**D-3400 Göttingen(DE)**

(54) **Neue Angucyclinone aus Streptomyceten, Verfahren zu ihrer Herstellung und ihre Vewendung.**

(57) Mit Hilfe eines Stammes aus der Gattung Streptomyces können neue Angucyclinone mit therapeutischer Wirkung hergestellt werden.

EP 0 339 442 A2

# Neue Angucyclinone aus Streptomyceten, Verfahren zu ihrer Herstellung und ihre Verwendung

Es ist bekannt, daß Streptomyces spec. unter herkömmlichen Kulturbedingungen ein Angucyclinon mit Namen Ochromycinon synthetisiert [Bowie J. H., Johnson A.W. Tetrahedron Letters 16, 1449 (1967)].

Es wurde nun überraschend gefunden, daß Streptomyces spec. DSM 4357 neben dem Ochromycinon neue Angucyclinone mit pharmakologischer, insbesondere antibiotischer Wirksamkeit bildet.

Die Erfindung betrifft somit:

1. Eine Verbindung der allgemeinen Formel I,

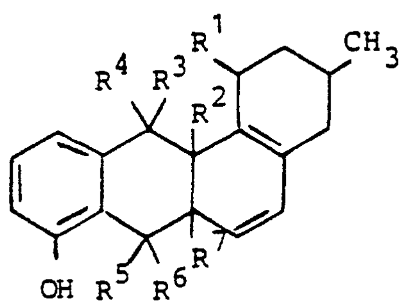

(I)

in der unabhängig voneinander
$R^1$ Hydroxyl oder eine Oxogruppe ist,
$R^2$ Wasserstoff
$R^3$ Hydroxyl und
$R^4$ Hydroxymethyl oder Wasserstoff oder
$R^3$ und $R^4$ zusammen eine Oxogruppe sind,
$R^5$ Hydroxymethyl oder Wasserstoff,
$R^6$ Hydroxyl oder
$R^5$ und $R^6$ zusammen eine Oxogruppe sind und
$R^7$ Hydroxyl oder Wasserstoff ist oder
$R^2$ und $R^7$ gemeinsam eine Doppelbindung bilden,
sowie die an den in der Formel I aufgeführten Hydroxylgruppen derivatisierten ($C_1$ bis $C_5$) Acyloxyverbindungen,
ausgenommen die Verbindung, in der $R^1$ sowie $R^3$ zusammen mit $R^4$ sowie $R^5$ zusammen mit $R^6$ eine Oxogruppe darstellen und $R^2$ und $R^7$ gemeinsam eine Doppelbindung bilden.

2. Ein Verfahren zur Herstellung der Verbindung der allgemeinen Formel I sowie deren ($C_1$ bis $C_5$) Acyloxyderivate, das dadurch gekennzeichnet ist, daß a) Streptomyces spec. DSM 4357 kultiviert wird, bis sich die Verbindung der allgemeinen Formel I im Kulturmedium anhäuft und b) gegebenenfalls die Verbindung isoliert und acyliert wird.

3. Die Verwendung der Verbindung der allgemeinen Formel I sowie deren ($C_1$ bis $C_5$) Acyloxyderivate als therapeutisch wirksamer Stoff.

Die Erfindung wird im folgenden detailliert beschrieben, insbesondere in ihren bevorzugten Ausführungsformen. Ferner wird die Erfindung in den Patentansprüchen definiert.

Die Verbindung der allgemeinen Formel I kann mit Hilfe von Streptomyces spec. DSM 4357 hergestellt werden. Der Stamm wurde am 15.1.1988 bei der Deutschen Sammlung von Mikroorganismen unter der angegebenen Nummer nach den Bedingungen des Budapester Vertrages hinterlegt.

Streptomyces spec. DSM 4357 hat folgende charakteristische Merkmale:

| | |
|---|---|
| Sporenfarbe:<br>Sporenkette:<br>Sporenoberfläche:<br>Melaninbildung: | grau<br>auseinandergezogene Spiralen<br>glatt<br>negativ |
| Pigmentbildung: | |
| Substratmycel:<br><br>Luftmycel:<br><br>Zucker- und<br>Zuckeralkoholverwertung: | Endo: negativ<br>Exo: violett<br>Endo: negative<br>Exo: negativ<br>Arabinose, Xylose, Rhamnose,<br>Raffinose, Mannit, Fructose,<br>Saccharose. |

Anstelle von Streptomyces spec. DSM 4357 können auch dessen Mutanten und Varianten verwendet werden, soweit sie eben die Verbindung der allgemeinen Formel I herstellen können. Solche Mutanten können in an sich bekannter Weise durch physikalische Mittel, beispielsweise Bestrahlung, wie mit Ultraviolett- oder Röntgenstrahlen oder chemische Mutagene, wie beispielsweise Ethylmethansulfonat (EMS), N-Methyl-N′-nitro-N-nitroso-guanidin (MNNG) oder 2-Hydroxy-4-methoxy-benzophenon (MOB) erzeugt werden.

Als bevorzugte Kohlenstoffquellen für die aerobe Fermentation eignen sich assimilierbare Kohlenhydrate und Zuckeralkohole, wie Glucose, Lactose oder D-Mannit, sowie kohlenhydrathaltige Naturprodukte, wie Malzextrakt. Als bevorzugte stickstoffhaltige Nährstoffe kommen in Betracht: Aminosäuren, Peptide und Proteine sowie deren Abbauprodukte, wie Peptone oder Tryptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. Außerdem kann die Nährlösung beispielsweise Chloride, Carbonate, Sulfate oder Phosphate der Alkali- oder Erdalkalimetalle, Eisen, Zink und Mangan als zusätzliche anorganische Salze enthalten.

Die Bildung der Verbindung der allgemeinen Formel I verläuft besonders gut in einer Nährlösung, die Glycerin in Konzentrationen von 0,5 bis 6 %, bevorzugt 2 - 4 %, sowie Sojamehl in Konzentrationen von 0,1 bis 4 %, bevorzugt 0,5 bis 2 % enthält, jeweils bezogen auf das Gewicht der gesamten Nährlösung.

Die Fermentation erfolgt aerob, also beispielsweise submers unter Schütteln oder Rühren in Schüttelkolben oder Fermentern, gegebenfalls unter Einführen von Luft oder Sauerstoff. Die Fermentation kann in einem Temperaturbereich von etwa 18 bis 40 °C, vorzugsweise bei etwa 25 bis 30 °C, insbesondere bei 28 bis 30 °C erfolgen. Man kultiviert den Mikroorganismus unter den genannten Bedingungen bis die stationäre Phase erreicht ist, etwa 60 bis 120 Stunden, bevorzugt 70 bis 75 Stunden.

Vorteilhaft kultiviert man in mehreren Stufen, d.h. man stellt zunächst eine oder mehrere Vorkulturen in einem flüssigen Nährmedium her, die dann in das eigentliche Produktionsmedium, die Hauptkultur, beispielsweise im Volumenverhältnis 1:10, überimpft werden. Die Vorkultur erhält man beispielsweise, indem man ein versportes Mycel in eine Nährlösung überimpft und etwa 48 bis 72 Stunden wachsen läßt. Das versporte Mycel kann erhalten werden, indem man den Stamm etwa 7 Tage auf einem festen oder flüssigen Nährboden, beispielsweise Hefe-Malz-Agar, wachsen läßt.

Der Fermentationsverlauf kann anhand des pH-Wertes der Kultur oder des Mycelvolumens, durch Dünnschichtchromatographie oder Ausprüfen der biologischen Aktivität überwacht werden.

Die Isolierung der Angucyclinone der allgemeinen Formel I aus dem Kulturmedium erfolgt nach bekannten Methoden unter Berücksichtigung der chemischen, physikalischen und biologischen Eigenschaften der Produkte. Zum Testen der Antibiotika-Konzentration im Kulturmedium oder in den einzelnen Isolierungsstufen kann die Dünnschichtchromatographie, beispielsweise auf Kieselgel mit Chloroform/Methanol als Laufmittel verwendet werden, wobei die Menge der gebildeten Antibiotika zweckmäßig mit einer Eichlösung verglichen wird.

Die Angucyclinone der allgemeinen Formal I liegen sowohl im Mycel als auch in der Kulturbrühe vor. Daher wird zweckmäßig für die Isolierung der Substanz beides Aufgearbeitet. Vorteilhaft wird vor der eigentlichen Aufarbeitung das Mycel von der Kulturbrühe getrennt, beispielsweise durch Filtrieren oder Zentrifugieren. Die Verbindung der allgemeinen Formel I kann dann aus dem Überstand bzw. Filtrat isoliert werden, zweckmäßig im pH-Bereich von 2 bis 8, vorzugsweise bei pH-Werten von 5 bis 7. Der Stoff kann mit herkömmlichen Mitteln, beispielsweise polaren Lösungsmitteln extrahiert werden, z. B. niedrigen Alkanolen. Es ist jedoch vorteilhaft, die Flüssigkeit über ein Adsorberharz, wie beispielsweise einen

Adsorber auf Polystyrolbasis zu geben. Die Elution kann dann mit einem polaren Lösungsmittel erfolgen, bevorzugt niedrige Alkanole wie z.B. Methanol, die möglicherweise noch mit Wasser vermischt werden. Aus dem Eluat kann das Lösungsmittel durch Destillation entfernt und der wäßrige, die Angucyclinone enthaltende Rückstand, getrocknet werden.

Die Angucyclinone der allgemeinen Formel I sind farblose amorphe Feststoffe, die gut in Methanol, Aceton, DMSO, Dioxan und Chloroform löslich sind, jedoch nicht in Wasser und Alkanen. Durch basenkatalysierte Acylierung der Hydroxylgruppen mit einem entsprechenden Säureanhydrid gelangt man zu den gewünschten ($C_1$ bis $C_5$)-Acyloxyderivaten.

Die Verbindung der allgemeinen Formel I sowie die ($C_1$ bis $C_5$)-Acyloxyderivate, bevorzugt ($C_1$ - $C_2$)-Acyloxyderivate, können entsprechend ihrer Stabilität in galenische Zubereitungen eingearbeitet werden. Die antibakterielle und antifungische Wirkung kann im Agar-Diffusionstest in vitro gezeigt werden. Die Angucyclinone weisen außerdem eine starke Wirkung gegen Protozoen, besonders gegen Trichomonas vaginalis auf.

In den folgenden Beispielen wird die Erfindung weitergehend erläutert. Prozentangaben beziehen sich, wie schon in der vorangegangenen Beschreibung, auf das Gewicht. Die im folgenden angegebenen Rf-Werte beziehen sich auf SilG/UV 254 + 366, 0,25 mm Schichtdicke, Fa. Macherey & Nagel.

**Beispiele:**

1. a) Herstellung einer Sporensuspension des Produzentenstammes:

100 ml Nährlösung (4 g Hefeextrakt, 10 g Malzextrakt, 4 g Glukose, 1 l Leitungswasser, pH-Wert vor dem Sterilisieren 7,3) in einem 500 ml Erlenmeyerkolben werden mit dem Stamm DSM 4357 beimpft und 72 Stunden bei 27° C und 120 UpM auf der rotierenden Schüttelmaschine inkubiert. Anschließend werden 20 ml Kulturflüssigkeit in einem 500 ml Erlenmeyerkolben mit dem Nährboden der obengenannten Zusammensetzung, dem 20 g Agar/l zur Verfestigung zugegeben wurde, gleichmäßig verteilt und dekantiert. Die Kulturen werden 10 bis 14 Tage bei 27° C inkubiert. Die nach dieser Zeit entstandenen Sporen eines Kolbens werden mit 500 ml entionisiertem Wasser, das einen Tropfen eines handelsüblichen nichtionischen Tensids (Triton X100, Fa. Serva) enthält, abgeschwemmt, sofort weiterverwendet oder bei -22° C aufbewahrt.

b) Herstellung einer Kultur bzw. Vorkultur des Produzentenstammes im Erlenmeyerkolben

Ein 500 ml Erlenmeyerkolben mit 100 ml einer Nährlösung der Zusammensetzung 2 % Fleischmehl, 10 % Malzextrakt, 1 % Calciumcarbonat und Wasser ad 100 % (ph 7,2 vor dem Autoklavieren) wird mit einer auf einem Schrägrörchen gezogenen Kultur oder mit 0,2 ml Sporensuspension angeimpft und auf einer Schüttelmaschine bei 120 UpM und 27° C inkubiert. Die maximale Antibiotikumproduktion ist nach 72 Stunden erreicht. Zum Animpfen von 10 und 100 l Fermentern genügt eine 48 Stunden alte Submerskultur (5 %) aus der gleichen Nährlösung.

2. Herstellung der Angucyclinone

Ein 10 l Fermenter wird unter folgenden Bedingungen betrieben:

| | |
|---|---|
| Nährmedium: | 30 g/l Glycerin<br>2 g/l Caseinprotein<br>1 g/l $K_2HPO_4$<br>1 g/l NaCl<br>0,5 g/l $MgSO_4 \bullet 7 H_2O$<br>5 ml Spurenelementlösung |
| Spurenelemente: | 3 g/l $CaCl_2 \bullet 2H_2O$<br>1 g/l $FeC_6O_7H_5$<br>0,2 g/l $MnSO_4$<br>0,1 g/l $ZnCl_2$<br>0,025 g/l $CuSO_4 \bullet 5H_2O$<br>0,02 g/l $Na_2B_4O_7 \bullet 10H_2O$<br>0,004 g/l $CoCl_2$<br>0,01 g/l $Na_2MoO_4 \bullet 2H_2O$<br>pH 7,2 |
| Inkubationszeit: | 72 Stunden |
| Inkubationstemperatur: | 30 °C |
| Rührergeschwindigkeit: | 250 UpM |
| Belüftung: | 4 l Luft/Min. |

Durch wiederholte Zugabe weniger Tropfen ethanolischer Polyollösung kann die Schaumentwicklung unterdrückt werden. Das Produktionsmaximum wird nach ca. 70 Stunden (pH = 5,3) erreicht. Die Ausbeuten liegen bei ca. 20 mg/l.

3. Isolierung der Angucyclinone

Nach der Fermentation von DSM 4357 wird die Kulturbrühe unter Zusatz von 2 % Celite als Filterhilfsmittel filtriert. Das Mycel wird mit Aceton extrahiert, die organische Phase eingedampft und der wäßrige Rückstand zum Kulturfiltrat gegeben. Das Kulturfiltrat wird über ein Adsorberharz auf Polystyrolbasis (XAD2 FA. Fluka) gegeben. Der Durchlauf wird verworfen und die Angucyclinone mit Methanol/$H_2O$ (80:20) eluiert. Das Eluat wird destilliert. Die Angucyclinone befinden sich im Destillationsrückstand.

4. Isolierung und Charakterisierung der Verbindung

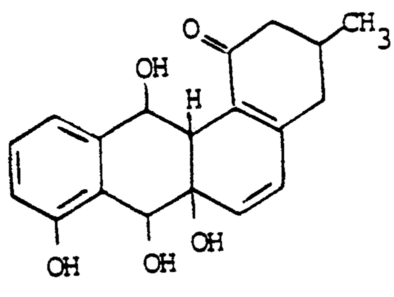

(Ia):

Die Verbindung Ia konnte aus dem Kulturfiltrat des Streptomycetenstammes DSM 4357 isoliert werden. Das Lyophilisat aus einer 150 l Fermentation wurde mit 10 g Kieselgel in wenig Laufmittel suspendiert, auf eine vorbereitete Kieselgel-Säule (10 x 25 cm, Kieselgel 0,04-0,063 mm) gegeben und mit Chloroform-Methanol (2 l, 20:1; 5 l Gradient bis 9:1) under Mitteldruck in l Fraktionen aufgetrennt:

| | |
|---|---|
| Fraktion 1: | 180 g einer Mischung weiterer Komponenten mit einem größeren Rf-Wert als Ia, |
| Fraktion 2: | 2,8 g, Rf = 0,58 [Chloroform-Methanol (9:1, v:v)]. |

Schmelzpunkt: 168 °C.

IR (KBr): $\nu$ = 3400, 3250, 2955, 2905, 2978, 1635, 1595, 1565, 1470, 1300, 1282, 1255, 1020, 1000, 790, 750 $cm^{-1}$.
UV ($CHCl_3$:$CH_3OH$ = 1:1): λ (1g ε) = 234 (2,4), 286 (2,8) nm.
$^1$H-NMR (200 MHz, DSMO-$D_6$, 35° C): δ = 9,29 (s; 1H, H/D), 7,08 (dd, J = 8,0 Hz; 1H), 6,77 (d, J = 8,0 Hz; 1H), 6,74 (d, J = 8,0 Hz; 1H), 6,24 (d, J = 9,0 Hz; 1H), 6,10 (d, J = 9,0 Hz; 1H), 5,77 (dd, J = 4,0 Hz, J = 9,0 Hz; 1H), 5,45 (s; 1H, H/d), 4,96 (d, J = 6,0 Hz; 1H, H/D), 4,68 (d, J = 6,0 Hz; 1H), 4,63 (d, J = 9,0 Hz; 1H, H/D), 3,16 (d, J = 4,0 Hz; 1H), 2,6 - 1,95 (m; 5H), 1,03 (d, J = 5,5 Hz; 3H) ppm.
$^{13}$C-NMR (200 MHz, DMSO-$d_6$): δ = 198,1 (C=O), 156,1 (C), 149,4 (C), 140,8 (C), 136,8 (CH), 128,8 (C), 127,8 (CH), 127,4 (CH), 123,1 (C), 120,5 (CH), 113,5 (CH), 71,1 (C), 67,6 (CH), 67,2 (CH), 48,5 (CH), 46,1 ($CH_2$), 37,5 ($CH_2$), 28,2 (CH), 20,3 ($CH_3$) ppm.
EI-MS (70 eV): m/e = 310 (M-$H_2O$, >1 %), 292 (M-2$H_2O$, 100 %), Hochauflösung: 292,1102 entspricht $C_{19}H_{15}O_3$ .
Summenformel: $C_{19}H_{20}O_5$ (328,3749 g $mol^{-1}$).

5. Herstellung und Charakterisierung der Di-O-acetyl- und Tri-O-acetyl-Verbindungen der Formel Ia

40 mg (0.12 mmol) Ia wurden einen Tag in 1 ml Pyridin und 1 ml Essigsäureanhydrid bei 4° C gerührt. Das Reaktionsgemisch versetzte man zweimal mit je 100 ml Toluol und dampfte das Lösungsmittel im Vakuum ab. Die säulenchromatographische Trennung (Säule: 2,5 x 25 cm) an Kieselgel (0.064 mm) in Chloroform ergab 2 Fraktionen:

| | |
|---|---|
| Fraktion 1: | 2,6 mg Tri-O-acetyl-Ia ( 5 %) |
| Fraktion 2: | 44,8 mg Di-O-acetyl-Ia (91 %). |

Di-O-acetyl-Ia:

Schmp. = 250° C.
Rf = 0.44 (Chloroform-Methanol = 9:1, v:v), 0.13 (Chloroform-Methanol = 98.2, v:v).
(auf SilG/UV 254+366; 0,25 mm Schichtdicke, Fa. Macherey & Nagel)
IR (KBr): $\nu$ = 3440, 2955, 2925, 2875, 1760, 1740, 1625, 1565, 1460, 1410, 1370, 1295, 1230, 1192, 1050, 1020 $cm^{-1}$.
UV ($CHCl_3$): λ (1g ε) = 295 (3,8) nm.
$^1$H-NMR (200 MHz, $CDCl_3$): δ = 7,5 - 7,4 (m; 2H), 7,12 -7,10 (m; 1H), 6,30 (s; 1H), 6,22 (d, J = 11,2 Hz; 1H), 6,17 (d, J = 9,5 Hz; 1H), 6,12 (dd, J = 4,0 Hz, J = 6,8 Hz; 1H), 4,57 (s; 1H, H/D), 4,15 (d, J = 6,8 Hz; 1H, H/D), 3,29 (m; 1H), 2,7 - 2,45 (m; 2H), 2,26 (s; 3H), 2,3 - 2,1 (m; 3H), 1,96 (s; 3H), 1,06 (d, J = 6.0 Hz; 3H) ppm.
$^{13}$C-NMR (200 MHz, $CDCl_3$): δ = 201,6 (C=O), 169,9 (C=O), 169,8 (C=O), 151,5 (C), 149,9 (C), 140,3 (C), 134,2 (CH), 130,1 (2xCH), 128,6 (CH), 127,2 (C), 124,0 (C), 122,5 (CH), 70,9 (C), 68,3 (CH), 67,6 (CH), 47,3 ($CH_2$), 39,3 (CH), 38,8 ($CH_2$), 29,1 (CH), 21,0 (2x$CH_3$), 20,9 ($CH_3$) ppm.
EI-MS (70 eV): m/e = 352 (M-$CH_3CO_2H$, 5 %), 334 (M-$CH_3CO_2H$-$H_2O$, 11 %), 292 (M-2$CH_3CO_2H$, 100 %).
Summenformel: $C_{23}H_{24}O_7$ (412,4516 g $mol^{-1}$).

Tri-O-acetyl-Ia

Schmp. = 98° C.
Rf = 0.88 (Chloroform-Methanol = 9:1, v:v), 0.23 (Chloroform-Methanol = 98:2, v:v).
(auf SilG/UV 254+366; 0,25 mm Schichtdicke, Fa. Macherey & Nagel)
$^1$H-NMR (200 MHz, $CDCl_3$): δ = 7,83 (d, J = 8,0 Hz; 1H), 7,75 (d, J = 4,5 Hz; 1H), 7,44 (dd, J = 8,0 Hz, J = 8,0 Hz; 1H), 7,14 (dd, J = 1,2 Hz, J = 8,0 Hz; 1H), 6,37 (d, J = 9,5 Hz; 1H), 6,34 (s; 1H, H/D), 6,22 (d, J = 9,5 Hz; 1H), 2,7 - 2,4 (m; 2H), 2,32 (s, 3H), 2,3 - 2,0 (m; 3H), 1,99 (s; 3H), 1,95 (s; 3H), 1,09 (d, J = 6,0 Hz; 3H) ppm.
EI-MS (70 eV): m/e = 394 (M-$CH_3CO_2H$, 6%), 334 (M-2$CH_3CO_2H$, 24 %), 292 (M-2$CH_3CO_2H$-$CH_3CO$, 100 %).
Summenformel: $C_{25}H_{26}O_8$ (454,488 g $mol^{-1}$).

6. Isolierung und Charakterisierung der Verbindung Ib

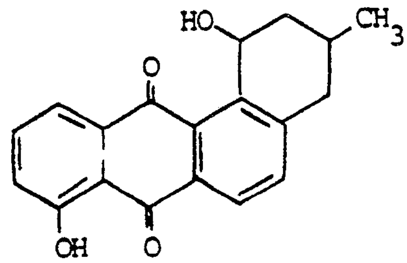

(Ib)

sowie

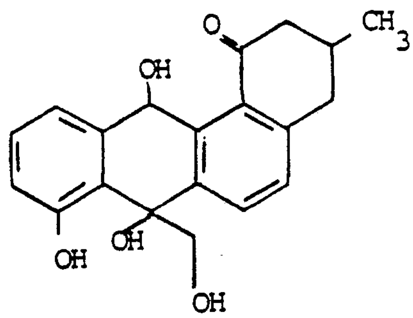

und

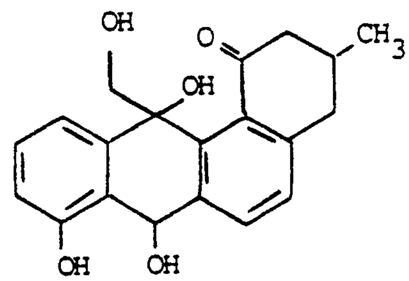

(Ic)

Aus dem Mycel des Streptomyces-Stammes DSM 4357 konnten die Verbindungen Ib und c isoliert werden. Das Mycel einer 150 l Fermentation wurde zweimal mit je 30 l Aceton extrahiert. Das vom Niederschlag abfiltrierte Lösungsmittel dampfte man bis zur wäßrigen Phase im Vakuum ein. Das Lyophilisat wurde in wenig Laufmittel suspendiert und auf einer Säule (10 x 20 cm; Kieselgel 0.04 - 0.063 mm) mit n-Hexan-Essigester (4 l, 4:1 2 l Gradient bis 3:7) unter Mitteldruck in 2 Fraktionen aufgetrennt:

| | |
|---|---|
| Fraktion 1: | 250 mg Ib Rf = 0.35 (n-Hexan-Essigester 4:1, v:v), |
| Fraktion 2: | 3,5 g Ic in Mischung mit weiteren Komponenten, mit kleineren Rf-Werten als Ib. |

Verbindung Ib

Rf = 0.35 (n-Hexan-Essigester 4:1, v:v).
IR (KBr): $\nu$ = 3450, 2950, 2920, 2860, 1700, 1660, 1630, 1580, 1560, 1450, 1370, 1315, 1275, 1250, 1160 $cm^{-1}$.
UV ($CH_3OH$): $\lambda$ = 217, 260, 402 nm.
$^1H$-NMR (200 MHz, $CDCl_3$): $\delta$ = 12,5 (s; 1H, H/D), 8,23 (d, J = 8,0 Hz; 1H), 7,79 (dd, J = 1,5 Hz, J = 8,0 Hz; 1H), 7,65 (dd, J = 8,0 Hz, J = 8,0 Hz; 1H), 7,53 (d, J = 8,0 Hz; 1H), 7,28 (dd, J = 1,5 Hz, J 8,0 Hz; 1H), 5,47 (ddd, J = 4,5 Hz, J = 8,0 Hz; 1H), 4,90 (d, J = 4,5 Hz; 1H, H/D), 2,86 (ddd, J = 2,5 Hz, J = 4,5 Hz, J = 17,5 Hz; 1H), 2,65 (dd, J = 10,5 Hz, J = 17,0 Hz; 1H), 2,39 (tdd, J = 2,8 Hz, J = 7,2 Hz, J = 13,0 Hz; 1H), 2,00 - 1,75 (m; 1H), 1,61 (ddd, J = 9,0 Hz, J = 11,8 Hz, J = 13,0 Hz; 1H), 1,13 (d, J = 7,0 Hz; 3H) ppm.
$^{13}C$-NMR (200 MHz, $CDCl_3$): $\delta$ = 188,2 (C=O), 187,0 (C=O), 161,8 (C), 147,0 (C), 143,4 (C), 136,7 (CH), 135,5 (CH), 134,6 (C), 133,8 (C), 132,3 (C), 126,5 (CH), 124,0 (CH), 120,1 (CH), 115,2 (C), 66,6 (CH), 40,5 ($CH_2$), 39,9 ($CH_2$), 27,5 (CH), 21,5 ($CH_3$) ppm.
EI-MS (70 eV): m/e = 308 (M, 100 %), 290 (M-$H_2O$, 5 %).
Summenformel: $C_{19}H_{16}O_4$ (308,3436 g $mol^{-1}$).

Die zweite Fraktion der säulenchromatographischen Trennung des Mycel-Rohproduktes, konnte durch eine weitere Trennung nach dem oben beschriebenen Verfahren in 3 Fraktionen aufgetrennt werden.

Verbindung Ic: Rf = 0.07 (n-Hexan-Essigester 4:1, v:v).

Verbindung Ic:

Schmp. = 212 °C.
Rf = 0.71 (Chloroform-Methanol = 9:1, v:v).
(auf SilG/UV 254+366; 0,25 mm Schichtdicke, Fa Macherey & Nagel)
IR (KBr): $\nu$ = 3280, 2960, 2875, 1683, 1630, 1590, 1500, 1467, 1350, 1280 $cm^{-1}$.
UV ($CH_3OH$): $\lambda$ (1g $\epsilon$) = 220 (4,1) 281 (3,7) nm.
$^1H$-NMR (200 MHz, Aceton-$d_6$): $\delta$ = 8,5 (m; 1H, H/D), 7,15 -6,95 (m; 3H), 6,85 - 6,7 (m; 2H), 5,89 (s; 1H), 5,22 (d, J = 15,0 Hz; 1H), 4,02 (d, J = 15,0 Hz; 1H), 2,99 (s; 1H, H/D), 2,93 (dd, J = 6,0 Hz, J = 16,5 Hz; 1H), 2,3 - 2,56 (m; 1H), 2,27 (dd, J = 11,0 Hz, J = 16,5 Hz; 1H), 2,27 (dd, J = 3,0 Hz, J = 12,5 Hz; 1H), 1,45 (dd, J = 12,0 Hz, J = 12,5 Hz; 1H), 1,15 (d, J = 7,0 Hz; 3H) ppm.
$^{13}C$-NMR (200 MHz, Aceton-$d_6$): $\delta$ = 200 (C=O), 154,9 (C), 150,0 (C), 144,5 (C), 139,5 (C), 129,1 (CH), 128,4 (CH), 126,4 (C), 126,1 (C), 124,6 (C), 120,1 (CH), 118,2 (CH), 115,5 (CH), 108,5 (C), 85,3 (CH), 59,5 ($CH_2$), 42,1 ($CH_2$), 34,7 ($CH_2$), 28,2 (CH), 22,1 ($CH_3$) ppm.
EI-MS (70 eV): m/e = 310 (M-$CH_2O$, 23 %), 292 (M-$CH_2OH$-$H_2O$, 15 %).
Summenformel: $C_{20}H_{20}O_5$ (340,3864 g $mol^{-1}$).

## Ansprüche

1. Verbindung der allgemeinen Formel I

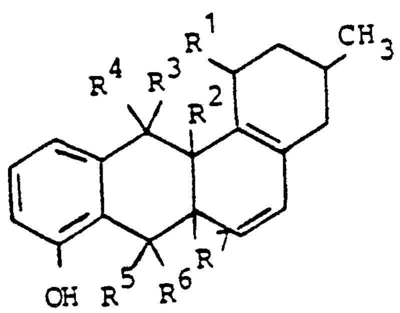

(I)

in der unabhängig voneinander
$R^1$ Hydroxyl oder eine Oxogruppe ist,
$R^2$ Wasserstoff
$R^3$ Hydroxyl und
$R^4$ Hydroxymethyl oder Wasserstoff oder
$R^3$ und $R^4$ zusammen eine Oxogruppe sind,
$R^5$ Hydroxymethyl oder Wasserstoff,
$R^6$ Hydroxyl oder
$R^5$ und $R^6$ zusammen eine Oxogruppe sind und
$R^7$ Hydroxyl oder Wasserstoff ist oder
$R^2$ und $R^7$ gemeinsam eine Doppelbindung bilden,
sowie die an den in der Formel I aufgeführten Hydroxylgruppen derivatisierten ($C_1$ bis $C_5$) Acyloxyverbindungen,
ausgenommen die Verbindung in der $R^1$ sowie $R^3$ zusammen mit und $R^4$ sowie $R^5$ zusammen mit $R^6$ eine Oxogruppe darstellen und $R^2$ und $R^7$ gemeinsam eine Doppelbindung bilden.

2. Verfahren zur Herstellung der Verbindung der allgemeinen Formel I sowie deren ($C_1$ bis $C_5$)-Acyloxyderivate nach Anspruch 1, dadurch gekennzeichnet, daß

a) Streptomyces spec. DSM 4357 oder seine Mutanten und Varianten kultiviert werden bis sich die Verbindung der allgemeinen Formel I in der Kultur anhäuft und

b) gegebenenfalls die Verbindung isoliert und acyliert wird.

3. Verfahren nach Anspruch 2, dadurch gekennzeichnet, daß der Mikroorganismus in einer Nährlösung kultiviert wird, die 0,5 bis 6 % Glycerin sowie 0,1 bis 4 % Sojamehl enthält, jeweils bezogen auf das Gewicht der gesamten Nährlösung.

4. Verfahren nach Anspruch 3, dadurch gekennzeichnet, daß die Nährlösung 2 bis 4 % Glycerin sowie 0,5 bis 2 % Sojamehl enthält.

5. Verfahren nach einem oder mehreren der Ansprüche 2 bis 4, dadurch gekennzeichnet, daß die Kultivierung in einem Temperaturbereich von etwa 18 bis 40 °C durchgeführt wird.

6. Verwendung der Verbindung der Formel I sowie deren ($C_1$ bis $C_5$)-Acyloxyderivate nach Anspruch 1 als therapeutisch wirksame Stoffe.

7. Verwendung der Verbindung nach Anspruch 6, dadurch gekennzeichnet, daß sie als Antibiotikum eingesetzt wird.

8. Verwendung nach Anspruch 7, dadurch gekennzeichnet, daß das Antibiotikum gegen Bakterien und Pilze sowie gegen Protozoen eingesetzt wird.

9. Streptomyces spec. DSM 4357 sowie seine Varianten und Mutanten, sofern sie die Verbindung der allgemeinen Formel I nach Anspruch 1 synthetisieren.

Patentansprüche für folgende Vertragsstaaten: ES, GR

1. Verfahren zur Herstellung der Verbindung der allgemeinen Formel I

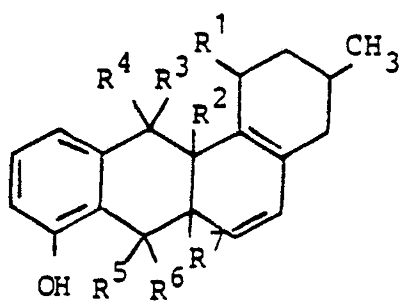

(I)

in der unabhängig voneinander
$R^1$ Hydroxyl oder eine Oxogruppe ist,
$R^2$ Wasserstoff
$R^3$ Hydroxyl und
$R^4$ Hydroxymethyl oder Wasserstoff oder
$R^3$ und $R^4$ zusammen eine Oxogruppe sind,
$R^5$ Hydroxymethyl oder Wasserstoff,
$R^6$ Hydroxyl oder
$R^5$ und $R^6$ zusammen eine Oxogruppe sind und
$R^7$ Hydroxyl oder Wasserstoff ist oder
$R^2$ und $R^7$ gemeinsam eine Doppelbindung bilden,
sowie die an den in der Formel I aufgeführten Hydroxylgruppen derivatisierten ($C_1$ bis $C_5$) Acyloxyverbindungen,
dadurch gekennzeichnet, daß

a) Streptomyces spec. DSM 4357 oder seine Mutanten und Varianten kultiviert werden bis sich die Verbindung der allgemeinen Formel I in der Kultur anhäuft und

b) gegebenenfalls die Verbindung isoliert und acyliert wird.

2. Verfahren nach Anspruch 1, dadurch gekennzeichnet, daß der Mikroorganismus in einer Nährlösung kultiviert wird, die 0,5 bis 6 % Glycerin sowie 0,1 bis 4 % Sojamehl enthält, jeweils bezogen auf das Gewicht der gesamten Nährlösung.

3. Verfahren nach Anspruch 2, dadurch gekennzeichnet, daß die Nährlösung 2 bis 4 % Glycerin sowie 0,5 bis 2 % Sojamehl enthält.

4. Verfahren nach einem oder mehreren der Ansprüche 1 bis 3, dadurch gekennzeichnet, daß die Kultivierung in einem Temperaturbereich von etwa 18 bis 40 °C durchgeführt wird.

5. Verwendung der Verbindung der Formel I sowie deren ($C_1$ bis $C_5$)-Acyloxyderivate nach Anspruch 1 als therapeutisch wirksame Stoffe.

6. Verwendung der Verbindung nach Anspruch 5, dadurch gekennzeichnet, daß sie als Antibiotikum eingesetzt wird.

7. Verwendung nach Anspruch 6, dadurch gekennzeichnet, daß das Antibiotikum gegen Bakterien und Pilze sowie gegen Protozoen eingesetzt wird.

8. Streptomyces spec. DSM 4357 sowie sein Varianten und Mutanten, sofern sie die Verbindung der allgemeinen Formel I nach Anspruch 1 synthetisieren.